Europäisches Patentamt

European Patent Office

Office européen des brevets

(19)

(11) Numéro de publication: **0 206 907**
**B1**

# FASCICULE DE BREVET EUROPÉEN

(12)

(45) Date de publication du fascicule du brevet:
**10.05.89**

(21) Numéro de dépôt: **86401271.1**

(22) Date de dépôt: **11.06.86**

(51) Int. Cl.⁴: **C 07 F 5/00,** C 07 F 15/00,
C 07 F 7/00, C 07 C 57/00,
C 07 C 51/00

(54) **Procédé de préparation d'une dispersion colloidale d'un composé de cation métallique en milieu organique et les sols obtenus.**

(30) Priorité: **20.06.85 FR 85709373**

(43) Date de publication de la demande:
**30.12.86 Bulletin 86/52**

(45) Mention de la délivrance du brevet:
**10.05.89 Bulletin 89/19**

(84) Etats contractants désignés:
**AT BE CH DE FR GB IT LI LU NL SE**

(56) Documents cité:
**EP-A-0 093 627**
**EP-A-0 097 563**
**EP-A-0 112 219**
**FR-A-2 172 797**
**FR-A-2 359 192**

(73) Titulaire: **RHONE- POULENC CHIMIE, 25, quai Paul Doumer, F-92408 Courbevoie Cédex (FR)**

(72) Inventeur: **Chane- Ching, Jean- Yves, 19, raritam avenue, Highland Park N.J. 08904 (US)**
Inventeur: **Fabre, Frédéric, Appartment 506 10, rue Louis Blanc, F-75010 - Paris (FR)**
Inventeur: **Herviou, Christian, 18, rue Nansouty, F-75014 - Paris (FR)**

(74) Mandataire: **Dutruc- Rosset, Marie- Claude, RHONE- POULENC INTERSERVICES Service Brevets Chimie 25, Quai Paul Doumer, F-92408 Courbevoie Cédex (FR)**

EP 0 206 907 B1

## Description

La présente invention a pour objet un procédé de prépartion d'une dispersion colloïdale d'un composé de cation métallique en milieu organique.

Plus précisément, l'invention concerne un procédé de préparation d'une dispersion colloïdale organique d'un composé d'un cation métallique à caractère acide dénommé par la suite cation $M^{n+}$ : $n+$ symbolise le degré d'oxydation du métal et est généralement égal à $+3$ ou $+4$.

Par composé de cation métallique, on entend un composé essentiellement à base de son hydroxyde métallique et d'un acide organique.

Par cation métallique à caractère acide, on désigne un cation dont l'hydroxyde métallique précipite à des faibles valeurs de pH de préférence à un pH inférieur à 4.

A titre de cations acide, on peut citer les cations des métaux suivants cérium, fer, titane, zirconium et étain.

L'invention vise tout particulièrement la fabrication de dispersions colloïdales d'un composé de cérium IV et/ou d'un composé de fer III, en milieu organique.

On connaît selon la demande française publiée sous le n° 2 359 192 des sels organiques de cérium solubles dans les solvants dont la caractéristique est d'avoir un rapport r entre le nombre d'équivalents acide et le nombre d'atomes de cérium compris entre 0,2 et 1; le nombre d'équivalents d'acide représente le nombre de molécules d'acide lorsque l'acide utilisé est monofonctionnel, et il faut doubler ou tripler ce nombre, dans le cas de diacides ou triacides et, plus généralement, le multiplier par le nombre de fonctions acide dans le cas d'un polyacide. Les composés du cérium ainsi proposés nécessitent une quantité d'acide beaucoup plus faible que la quantité utilisée antérieurement pour obtenir la même efficacité; on peut également obtenir des solutions de concentration élevée en métal pouvant aller jusqu'à 500 g/l; les solutions obtenues restent fluides et peuvent être manipulées sans aucune difficulté tout en restant parfaitement solubles dans les milieux d'hydrocarbures.

L'acide organique peut être $RC\ OOH$, $R\ SO_3H$, $RO\ SO_3H$, $RO\ PO_3\ H_2$ ou $(RO)_2\ P{=}_2H$, R étant un radical hydrocarboné ayant au moins 7 atomes de carbone. Le radical acide organique peut être un radical aliphatique linéaire ou ramifié ou un radical cycloaliphatique éventuellement substitué par un radical alcoyle ou un radical aromatique éventuellement substitué par un radical alcoyle. Les sels de cérium de ces acides organiques peuvent, en plus, contenir au moins un autre métal de terres rares et ce, jusqu'à 25 % de la teneur totale en métaux de terres rares, y compris le cérium. On peut obtenir des compositions fournies sous la forme de solution dans un solvant organique, d'un sel de cérium d'acide organique ou d'un mélange de sels contenant plus de 200 g de cérium par litre de composition.

Le procédé de préparation de ces sels de cérium d'acide organique ou de leurs mélanges consiste à faire réagir dans un milieu organique ou hydroorganique, l'acide organique et l'hydroxyde de cérium Ce $(OH)_3$ fraîchement préparé de sorte que les sels de cérium d'acide organique obtenus aient un rapport r compris entre 0,2 et 1. La réaction s'effectue de préférence en chauffant et le milieu organique est de préférence un hydrocarbure. Après plusieurs heures, une partie de l'eau qui s'est formée durant la réaction décante spontanément. Après réaction, il est possible d'aider à la séparation de l'eau formée à partir du milieu réactionnel en ajoutant un tiers solvant tel qu'un glycol, un alcool ou un alcoylglycol. On peut ajuster la concentration de la solution ainsi obtenue en ajoutant un hydrocarbure approprié.

Dans les exemples, l'hydroxyde de cérium Ce $(OH)_3$ est obtenu par précipitation du nitrate de cérium par l'ammoniaque. On lave le précipité à l'eau jusqu'à disparition de l'ion nitrate; ensuite, on filtre le précipité jusqu'à ce qu'il ne contienne plus que 15 % d'eau. On fait réagir à 80°C l'hydroxyde de cérium avec 130 g d'acide oléique technique dans du white-spirit. Après quatre heures on ajoute du glycol, on élimine l'eau qui s'est séparée et, ensuite, on ajoute du butyl-glycol, puis du white-spirit pour constituer la solution finale.

Il a également été proposé selon la demande européenne publiée sous le n° 0 097 563 un procédé de préparation de dispersions colloïdales de dioxyde cérique dans des liquides organiques inertes qui consiste:

[1]     à chauffer entre 60°C et 200°C,

(a) du dioxyde cérique comprenant du nitrate d'ammonium en une quantité représentant entre 3 % et 14 % du poids de dioxyde cérique et un membre choisi dans le groupe formé par l'eau, le méthanol, l'acide acétique et leurs mélanges en une quantité d'au moins 10 g par mole de $CeO_2$,

(b) un acide organique ayant environ 10 à environ 40 atomes de carbone tout particulièrement l'acide oléique,

(c) un liquide organique choisi dans le groupe formé par les hydrocarbures aliphatiques, cycloaliphatiques et aromatiques, les éthers ou cétones aliphatiques et cycloaliphatiques,

[2]     puis à éliminer l'eau, le méthanol, l'acide acétique libéré pendant le chauffage et à séparer toutes les particules solides non dissoutes.

On obtient une dispersion colloïdale de dioxyde cérique dans un milieu organique le dioxyde cérique se trouvant sous la forme d'un complexe qui résulte de l'association physique $CeO_2$ - acide organique.

Il ressort de l'analyse de l'art antérieur que les procédés permettant d'obtenir des sols organiques de dioxyde cérique font appel, à titre de matière première, à du dioxyde cérique hydraté qui est préparé le plus souvent, par oxydation et précipitation par une base d'une solution de sel de cérium III, puis séparation du précipité obtenu.

2

Un des buts de la présente invention est de fournir un procédé permettant d'obtenir directement une dispersion colloïdale d'un composé de cérium IV en milieu organique, à partir d'une solution aqueuse d'un sel de cérium IV sans passage par l'étape de précipitation et séparation du dioxyde de cérium hydraté.

En ce qui concerne les sels organiques de fer, on peut citer la demande de brevet française 2 172 797 qui décrit des sels organiques du fer solubles en milieu organique constitués par un complexe d'un acide organique ou organométalloïdique et du fer ferrique dans lequel le rapport R du nombre d'équivalents d'acide organique au nombre d'atomes de fer ferrique est inférieur à 3.

Le procédé de fabrication desdits composés est caractérisé en ce qu'il consiste à faire réagir, sur de l'hydroxyde ferrique solide fraîchement préparé, un ou plusieurs des acides précédemment mentionnés dans des proportions relatives telles que le rapport du nombre de molécules d'acide (ou d'équivalents dans le cas de diacides ou de polyacides) au nombre d'atomes de fer ferrique soit inférieur à 3/1 et de préférence compris entre 1/8 et 2: cet hydroxyde ferrique peut être préparé in situ dans le milieu réactionnel à partir d'un sel ferrique ou bien il peut avoir été préparé préalablement dans une étape distincte, mais très peu de temps avant l'étape de formation du sel organo soluble. Selon une variante on peut partir d'un sel ferreux soluble dans l'eau que l'on transforme préalablement en sel ferrique; on peut aussi précipiter l'hydroxyde ferreux à partir d'une solution aqueuse de sel ferreux et transformer ensuite cet hydroxyde en hydroxyde ferrique par un traitement d'oxydation, par exemple par l'oxygène de l'air ou par un oxydant tel que l'eau oxygénée ou le chlore.

Un des buts de l'invention est de disposer d'un procédé permettant de s'affranchir des diverses contraintes de ce procédé:

- nécessité d'élaboration d'un hydroxyde ferrique solide fraîchement préparé dans le cas d'obtention d'un sel organique à partir d'un hydroxyde solide,
- nécessité d'un contrôle rigoureux des paramètres de marche de fabrication (agitation, conditions de mélange) dans le cas d'une élaboration de sel organique avec utilisation d'un hydroxyde ferrique préparé in situ.

Il est également connu selon la demande EP-A-0 112 219 de préparer des compositions organométalliques mixtes entre les éléments du groupe des lanthanides et du manganèse ou des éléments du groupe du fer. Toutefois, leur procédé de préparation fait également appel aux hydroxydes des métaux précités.

Enfin un autre but de l'invention est de fournir un procédé de préparation d'une dispersion colloïdale organique convenant aussi bien au composé de cérium IV qu'au composé de fer III ou de tout autre cation métallique à caractère acide.

Il a maintenant été trouvé et c'est ce qui fait l'objet de la présente invention un procédé de préparation d'une dispersion colloïdale d'un composé d'un cation métallique à caractère acide en milieu organique caractérisé par le fait qu'il consiste:

- à mettre en contact:
  (a) une phase aqueuse constituée par au moins une dispersion colloïdale d'au moins un composé dudit cation $M^{n+}$ en milieu aqueux sursaturé en ions $OH^-$ obtenue par réaction d'un sel du cation $M^{n+}$ et d'une base,
  (b) une phase organique comprenant un acide organique et un milieu liquide organique ou solvant
- puis à séparer la phase aqueuse et la phase organique.

La caractéristique du procédé de l'invention est d'obtenir un sol organique expression qui désigne la dispersion du composé du cation $M^{n+}$ en milieu organique, par transfert dudit composé en phase organique, à partir d'une phase aqueuse constituée par le composé du cation $M^{n+}$ qui se trouve sous forme colloïdale dans un milieu aqueux sursaturé en ions $OH^-$.

On définit l'état de sursaturation comme étant une zone de pH où l'on trouve le composé du cation $M^{n+}$ sous forme colloïdale alors que l'on aurait dû s'attendre, compte-tenu du produit de solubilité de l'hydroxyde métallique, à la précipitation dudit hydroxyde. Cet état de sursaturation est atteint lorsque l'on introduit une quantité de base telle qu'elle représente de 60 à 95 % en moles de la quantité de base théorique nécessaire à la neutralisation du cation $M^{n+}$ présent dans le milieu réactionnel pour obtenir $M(OH)_n$.

On désigne par sol aqueux, la dispersion colloïdale du composé du cation $M^{n+}$ en milieu aqueux qui constitue la matière première de base du procédé de l'invention.

Afin de conduire de manière satisfaisante le procédé de l'invention, il est souhaitable que le sol aqueux de départ satisfasse aux exigences suivantes:

- le taux de métal M sous forme colloïdale doit être aussi élevé que possible et de préférence supérieur ou égal à 95 %,
- la concentration du sol aqueux en composé de cation $M^{n+}$ doit être suffisante et se situer de préférence entre 0,1 et 3 moles/litre,
- le sol aqueux doit présenter de bonnes propriétés de stabilité thermique et ne pas floculer à la température de réaction qui est supérieure à 60°C et varie le plus souvent entre 80 et 100°C.

Dans la première étape du procédé, on réalise la préparation d'au moins une dispersion colloïdale, d'au moins un composé d'un cation $M^{n+}$ en milieu aqueux sursaturé en ions OH⁻ en faisant réagir une solution aqueuse d'au moins un sel du cation $M^{n+}$ avec une base dans les conditions définies ci-après.

Conformément au procédé de l'invention, on peut mettre en oeuvre une dispersion aqueuse colloïdale d'un composé d'un cation $M^{n+}$, un mélange de deux ou plusieurs dispersions aqueuses colloïdales de différents composés d'un cation $M^{n+}$ une dispersion aqueuse colloïdale d'un composé de différents cations $M^{n+}$.

On donne ci-après une voie d'accès à une dispersion colloïdale aqueuse d'un composé d'un cation $M^{n+}$ : $M^{n+}$ symbolisant le cérium IV ou le fer III.

Pour la préparation de la dispersion colloïdale aqueuse d'un composé de cérium IV, objet de la demande EP-A-0 208 580, on peut faire appel à une solution aqueuse de nitrate cérique ou une solution aqueuse de nitrate céri-ammoniacal. Ladite solution peut contenir sans inconvénient du cérium à l'état céreux mais il est souhaitable qu'elle contienne au moins 85 % de cérium IV.

La solution de sel de cérium est choisie de telle sorte qu'elle ne contienne pas d'impuretés qui puissent se retrouver, dans le produit final. Notamment, il est préférable qu'elle soit exempte d'anions covalents à caractère coagulant tel que sulfates, etc. Cependant, on peut en tolérer de faibles quantités. Par exemple, lesdits anions peuvent représenter jusqu'à 5 % en poids du sel de cérium exprimé en $CeO_2$.

La concentration de la solution de sel de cérium peut varier entre 0,1 et 3 moles par litre. Il peut être intéressant pour des questions de productivité d'appareillage, de faire appel à une solution concentrée de sel de cérium IV: une concentration comprise entre 1,25 et 2 moles par litre est préférée.

La solution aqueuse du sel de cérium IV présente généralement une certaine acidité initiale et peut avoir une normalité variant entre 0,1 N et 4 N. La concentration en ions H+ n'est pas critique. Il est souhaitable qu'elle se situe entre 0,1 N et 1 N.

La solution de nitrate cérique obtenue selon le procédé d'oxydation électrolytique d'une solution de nitrate céreux et qui est décrite dans la demande de brevet française FR-A-2 570 087 (n° 8 413 641) constitue une matière première de choix.

La solution basique utilisée peut être notamment une solution aqueuse d'ammoniaque, de soude ou de potasse. On peut également faire appel à l'ammoniac gazeux. Selon l'invention, on met en oeuvre de préférence une solution d'ammoniaque.

La normalité de la solution basique mise en oeuvre n'est pas un facteur critique selon l'invention, elle peut varier dans de larges limites, par exemple, entre 1 et 11 N mais il est préférable, pour obtenir des solutions concentrées en cérium IV, de faire appel d'abord à une solution dont la concentration varie entre 5 et 11 N puis à une solution plus diluée par exemple de 1 à 5 N.

La proportion entre la solution basique et la solution de sel de cérium IV doit être telle que le taux de sursaturation soit supérieur à 3 et inférieur à 4.

On définit le taux de sursaturation r par l'équation suivante:

$$r = \frac{n3 - n2}{n1}$$

dans laquelle:
- n1 représente le nombre de moles de Ce IV présentes dans la dispersion colloïdale finale,
- n2 représente le nombre de moles OH⁻ nécessaires pour neutraliser l'acidité apportée par la solution aqueuse de sel de cérium IV,
- n3 représente le nombre total de moles OH⁻ apportées par l'addition de la base.

Le taux de sursaturation reflète l'état colloïdal du cérium IV:

- avec r = 4, le cérium IV précipite sous forme gélatineuse
- avec r = 0, le cérium IV est sous forme ionique
- avec 0 < r < 4,0, le cérium IV est sous forme ionique et/ou colloïdale.

La demanderesse a trouvé que l'obtention d'une bonne dispersion des colloïdes du composé de cérium IV dans la phase organique était liée à l'état colloïdal du composé de cérium IV dans la phase aqueuse.

On choisit selon l'invention un taux de sursaturation supérieur à 3 et inférieur ou égal à 3,8 pour une concentration finale en cérium IV de la dispersion colloïdale obtenue variant entre 0,1 M (soit 17 g/l de $CeO_2$) et 2 M (soit 344 g/l de $CeO_2$). D'une manière préférentielle, le taux de sursaturation est supérieur ou égal à 3,4 et inférieur ou égal à 3,8 pour une concentration finale en cérium IV de ladite dispersion variant entre 0,5 M (soit 86 g/l de $CeO_2$) et 1,2 M (soit 206 g/l).

D'une manière pratique, pour obtenir un taux de sursaturation désiré r choisi dans l'intervalle précité pour une concentration donnée en Ce IV dans la dispersion colloïdale finale, on ajuste la concentration de la solution basique de telle sorte qu'elle satisfasse à l'équation suivante:

$$[OH^-] = \frac{(n_1 \cdot r + n_2)[Ce\ IV]_i\ [Ce\ IV]_f}{n_1\ ([Ce\ IV]_i - [Ce\ IV]_f)}$$

- $[OH^-]$ représente la concentration en moles/litre de la solution basique,
- $[Ce\ IV]_f$ représente la concentration en ce IV en moles/litre de la dispersion colloïdale finale,
- $[Ce\ IV]_i$ représente la concentration en Ce IV en moles/litre de la solution aqueuse de sel de cérium IV,

4

- n₁ et n₂ sont déterminés par dosage classique de la solution aqueuse du sel de cérium IV:
. n₁ par titrage potentiométrique à l'aide d'une solution d'un sel ferreux,
. n₂ par titrage acido-basique après complexation du cérium à l'aide d'ions oxalate.

On peut faire correspondre à un taux de sursaturation donné, une quantité de base introduite, exprimée en pourcentage en moles de la quantité de base théorique nécessaire à la neutralisation complète du cérium IV présent dans le milieu réactionnel, pour obtenir $Ce(OH)_4$.

A un taux de sursaturation supérieur à 3 et inférieur à 4, correspond une quantité molaire de base introduite supérieure à 75 % et inférieure à 100 % de la quantité théorique.

A titre d'exemples, on mentionne qu'à des taux de sursaturation de 3,5 et 3,8 correspondent respectivement des quantités molaires de base introduites représentant 87,5 et 95 % des quantités théoriques.

Un mode d'obtention du taux de sursaturation consiste à contrôler le pH du milieu réactionnel.

L'obtention d'un taux de sursaturation inférieur ou égal à 3,8 correspond à un pH final de la dispersion colloïdale du composé de cérium IV inférieur ou égal à 3.

On précisera à titre d'exemples, que dans le cas d'une solution de nitrate cérique ayant une concentration telle que la concentration finale du ce IV dans la dispersion colloïdale obtenue soit de 0,7 M, on obtient respectivement les domaines de taux de sursaturation suivants correspondants aux domaines de pH définis ci-après:

$$0,2 < pH < 0,7 \qquad 3 < r < 3,3$$
$$0,7 < pH < 2,7 \qquad 3,3 < r < 3,7$$

La réaction entre la solution aqueuse de sel de cérium IV et la base mises en oeuvre dans les quantités définies précédemment est effectuée à une température qui peut se situer entre 0°C et 60°C mais de préférence à la température ambiante (le plus souvent 15 à 25°C).

On peut réaliser le mélange des réactifs précités selon plusieurs variantes. Par exemple, on peut faire le mélange simultané, sous agitation, de la solution aqueuse du sel de cérium IV et de la solution basique ou bien additionner, en continu ou en une seule fois, la base dans la solution aqueuse du sel de cérium IV et inversement.

Quel que soit l'ordre d'introduction des réactifs, on obtient une dispersion colloïdale d'un composé de cérium IV en milieu aqueux que l'on dénommera également par l'expression "sol aqueux".

Il est à noter que le composé de cérium IV se trouve sous la forme d'une dispersion colloïdale dans l'eau ce qui signifie que ledit composé a des particules de dimensions colloïdales mais ceci n'exclut pas la présence de ce IV sous forme ionique. Cependant, il est préférable que le taux de cérium sous forme colloïdale soit aussi élevé que possible et soit de préférence supérieur ou égal à 95 %.

Le composé de cérium IV sous forme colloïdale en milieu aqueux répond à la formule chimique suivante (I):

$$Ce(OH)_{4-x}(NO_3)_x \qquad \qquad (I)$$

dans laquelle x varie entre 0,3 et 0,7.

On peut toutefois observer la présence d'ions ammonium adsorbés sur les particules colloïdales.

Le sol aqueux obtenu présente une concentration en cérium IV qui n'est pas critique et peut donc varier entre 0,1 et 2 moles/litre.

Il contient également une forte concentration du sel de base qui se situe entre 0,3 et 8,0 moles/litre.

La densité des colloïdes est mesurée sur la dispersion colloïdale par détermination de la masse moléculaire à l'aide de la méthode de diffusion classique de la lumière et par corrélation avec le diamètre hydrodynamique défini selon la méthode de diffusion quasi-élastique de la lumière.

La densité des colloïdes est toujours inférieure à celle de $CeO_2$ (d = 7,2). Elle varie entre 3,5 et 6,0 et augmente au fur et à mesure que le taux de sursaturation croit.

La taille des colloïdes est définie par la mesure du diamètre hydrodynamique des colloïdes déterminé par diffusion quasi-élastique de la lumière selon la méthode décrite par Michael L. McConnell dans Analytical Chemistry Vol. 53 n° 8 1007 A (1981): ledit diamètre peut varier entre 5 nm (50 Å) et 40 nm (400 Å).

En ce qui concerne la préparation d'une dispersion colloïdale aqueuse d'un composé de fer III, on peut avoir recours à toute solution aqueuse de sel ferrique et notamment à une solution de chlorure ou de nitrate ferrique.

La solution de sel de fer est choisie de telle sorte qu'elle ne contienne pas plus de 5 % d'anions à caractère coagulant.

La concentration de la solution de sel de fer peut varier entre 0,1 et 3 moles par litre: une concentration comprise entre 0,5 et 1,5 moles par litre est préférée.

La solution aqueuse de sel de fer présente généralement une certaine acidité initiale qui ne présente aucun caractère critique: elle peut avoir une normalité variant entre 0,01 N et 2 N.

Le fer peut être introduit dans le milieu réactionnel à l'état ferreux, et on l'oxyde à l'état ferrique en ajoutant au mélange réactionnel un agent oxydant compatible avec ce milieu. Parmi les agents oxydants pouvant convenir, on peut citer notamment des solutions de perchlorate, de chlorure, d'eau oxygénée, ou l'air, l'oxygène, l'ozone. On peut également oxyder le fer par voie électrochimique.

On utilise de préférence l'eau oxygénée.

La proportion d'agent oxydant par rapport au fer ferreux à oxyder peut varier dans de larges limites. Elle est en général supérieure à la stoechiométrie et correspond, de préférence, à un excès compris entre 10 et 40 %.

La solution basique utilisée est semblable à celle décrite précédemment tant du point de vue de sa nature, de sa concentration que de sa mise en oeuvre.

La proportion entre la solution basique et la solution de fer est telle que le taux de sursaturation soit supérieur à 2,2 et inférieur à 2,7.

D'une manière pratique, pour obtenir un taux de sursaturation désiré, on peut ajuster la concentration de la solution basique en reprenant l'équation définie pour le cérium.

On précise à titre d'exemple que l'on peut également obtenir un taux de sursaturation compris entre 2,2 et 2,6 en contrôlant le pH du milieu réactionnel entre 1,7 et 2,0 pour une concentration finale en $Fe^{3+}$ de 0,5 mole/litre.

La réaction entre la solution aqueuse de sel de fer et la base peut être effectuée dans les mêmes conditions que celles décrites pour la préparation de la dispersion colloïdale aqueuse du composé de cérium IV. D'une manière préférentielle, on peut faire subir au mélange réactionnel, un traitement thermique entre 15 et 80°C pendant 5 minutes à 8 heures.

On obtient, une dispersion colloïdale d'un composé de fer III en milieu aqueux avec une taille de colloïdes pouvant varier entre 10 nm (100 Å) et 70 nm (700 Å).

Selon une première variante du procédé de l'invention, on peut mettre en oeuvre deux ou plusieurs dispersions colloïdales aqueuses de différents composés d'un cation $M^{n+}$.

C'est ainsi que l'on peut partir d'une dispersion colloïdale d'un composé de cérium IV et d'une dispersion colloïdale d'un composé de fer III préparées toutes deux par voie séparée.

La proportion entre lesdites dispersions peut être telle que l'on obtienne un mélange contenant:

- de 15 à 85 % d'un composé de cérium IV exprimé en poids de cérium rapporté aux poids totaux des métaux,
- de 15 à 85 % d'un composé de fer III exprimé en poids de fer rapporté aux poids totaux des métaux.

Selon une autre variante du procédé de l'invention on peut faire appel à une dispersion colloïdale aqueuse d'un composé de différents cations $M^{n+}$.

On prépare une dispersion colloïdale aqueuse d'un composé métallique mixte comprenant différents cations à caractère acide.

Le procédé d'obtention de ladite dispersion consiste à faire réagir une solution aqueuse d'au moins deux sels de métal M avec une base en une quantité de base telle qu'elle représente de 60 à 95 % en moles de la quantité de base théorique nécessaire à la neutralisation complète des cations $M^{n+}$ présents dans le milieu réactionnel pour obtenir $M(OH)_n$.

Les caractéristiques des réactifs et les conditions de mise en oeuvre correspondent à ce qui a été décrit précédemment.

On met en oeuvre au moins deux métaux M sous la même forme de préférence sous forme nitrate.

Le protocole opératoire peut différer selon que les métaux sont ou non tous sous forme de solution aqueuse.

On peut mélanger les solutions aqueuses d'au moins deux sels de métaux M puis procéder à l'addition de la base.

On peut également introduire un ou plusieurs des sels de métal M sous forme solide (anhydre ou hydratée) dans la solution aqueuse du sel d'au moins un autre métal M et ensuite ajouter la base.

On obtient une dispersion colloïdale d'un composé intermétallique en milieu aqueux qui présente des colloïdes dont le diamètre hydrodynamique peut varier entre 5 nm (50 Å) et 200 nm (2000 Å).

Selon le procédé de l'invention, on met en contact une phase aqueuse constituée par la ou les dispersions colloïdales précédemment décrites et une phase organique comprenant un milieu liquide organique ou solvant et un acide organique.

Le milieu liquide organique utilisé dans le procédé de l'invention peut être un hydrocarbure aliphatique ou cycloaliphatique inerte ou leur mélange tel que par exemple des essences minérales ou de pétrole, éthers minéraux ou de pétrole pouvant contenir également des composantes aromatiques. Les exemples comprennent l'hexane, l'heptane, l'octane, le nonane, le décane, le cyclohexane, le cyclopentane, le cycloheptane et les naphtènes liquides. Les solvants aromatiques tels que le benzène, le toluène, l'éthylbenzène et les xylènes conviennent également ainsi que les coupes pétrolières du type Solvesso (marque déposée par la Société EXXON) notamment le Solvesso 100 qui contient essentiellement un mélange de méthyléthyl et triméthylbenzène et le Solvesso 150 qui renferme un mélange d'alkylbenzènes en particulier de diméthyléthylbenzène et de tétraméthylbenzène.

On peut mettre en oeuvre également des hydrocarbures chlorés tels que le chloro- ou dichlorobenzène et le chlorotoluène aussi bien que des éthers aliphatiques et cycloaliphatiques tels que l'éther de diisopropyle, l'éther de dibutyle et les cétones aliphatiques et cycloaliphatiques telles que la méthylisobutylcétone, la diisobutylcétone, l'oxyde de mésityle.

On choisira le liquide organique ou système solvant en tenant compte de l'acide organique solubilisant utilisé, de la température de chauffage et de l'application finale de la solution ou dispersion colloïdale. Dans certains cas, il est préférable d'employer un mélange de solvants. La quantité de liquide ou solvant détermine évidemment la concentration finale. Il est plus économique et plus commode de préparer des dispersions plus concentrées qui pourront être diluées plus tard, lors de leur emploi. C'est pour cette raison que la quantité de solvant n'est pas critique.

Il peut être avantageux d'ajouter dans la phase organique un agent promoteur dont la fonction est d'accélérer le transfert des colloïdes de la phase aqueuse à la phase organique et d'améliorer la stabilité des sols organiques obtenus. A titre d'agents promoteurs, on peut utiliser les composés à fonction alcool et tout particulièrement des alcools aliphatiques linéaires ou ramifiés ayant de 6 à 12 atomes de carbone.

Comme exemples spécifiques, on peut citer l'éthyl-2 hexanol, le décanol, le dodécanol ou un mélange d'entre eux.

La proportion dudit agent dans la phase organique n'est pas critique et peut varier dans de larges limites.

Toutefois une proportion comprise entre 2 et 15 % en poids convient généralement bien.

Pour ce qui est de l'acide organique, on peut mettre en oeuvre des acides carboxyliques aliphatiques, des acides sulfoniques aliphatiques, des acides phosphoniques aliphatiques, des acides alcoylarylsulfoniques et des acides alcoylarylphosphoniques possédant environ de 10 à environ 40 atomes de carbone, qu'ils soient naturels ou synthétiques. On peut les utiliser seuls ou en mélange entre eux. A titre d'exemples, on peut citer les acides gras de tallöl, d'huile de coco, de soja, de suif, d'huile de lin, l'acide oléique, l'acide linoléique, l'acide stéarique, l'acide isostéarique, l'acide pelargonique, l'acide caprique, l'acide laurique, l'acide myristique, l'acide dodécylbenzènesulfonique, l'acide éthyl-2 hexoïque, l'acide naphténique, l'acide hexoïque, l'acide toluène-sulfonique, l'acide toluène-phosphonique, l'acide lauryl-sulfonique, l'acide lauryl-phosphonique, l'acide palmityl-sulfonique et l'acide palmityl-phosphonique. D'une manière préférentielle, on utilise l'acide oléique ou les acides alcoylaryl-sulfoniques.

La quantité d'acide organique mise en oeuvre exprimée en nombre de moles d'acide par mole du composé d'un cation $M^{n+}$ peut varier dans de larges limites entre 0,25 et 1 mole par mole de $M_2O_n$. La borne supérieure ne présente pas de caractère critique mais il n'est pas nécessaire de mettre en jeu davantage d'acide. D'une manière préférentielle, l'acide organique est mis en oeuvre à raison de 0,25 à 0,8 mole par mole de $M_2O_n$.

Dans la phase organique, la proportion entre le solvant organique et l'acide organique n'est pas critique. Le rapport pondéral entre le solvant organique et l'acide organique est choisi, de préférence, entre 0,3 et 2,0.

L'ordre d'introduction des différents réactifs est indifférent. On peut effectuer le mélange simultané de la ou des dispersions aqueuses colloïdales, de l'acide organique, du solvant organique et éventuellement de l'agent promoteur. On peut également faire le prémélange de l'acide organique, du solvant organique et éventuellement de l'agent promoteur qui constituent la phase organique.

La température du milieu réactionnel est choisie préférentiellement dans un intervalle allant de 60°C à 150°C.

Dans certains cas, en raison de la volatilité du solvant organique il y a lieu de condenser ses vapeurs par refroidissement à une température inférieure à son point d'ébullition.

D'une manière préférentielle, on travaille à une température comprise entre 80 et 100°C.

On maintient le mélange réactionnel sous agitation pendant toute la durée du chauffage qui peut être de moins d'une heure à environ 24 heures, de préférence entre 2 heures et 6 heures.

Au bout du temps de chauffage précité, on arrête le chauffage. On note la présence de deux phases: une phase organique contenant en dispersion, le complexe métallique - acide organique et une phase aqueuse contenant le sel de la base.

On sépare ensuite la phase organique et la phase aqueuse selon les techniques classiques de séparation: décantation, centrifugation etc..

Conformément à la présente invention, on obtient des dispersions organiques colloïdales d'un composé d'un cation métallique $M^{n+}$ contenant un ou plusieurs cations $M^{n+}$ dont la taille des colloïdes peut être très variable en jouant sur certains paramètres notamment le diamètre des dispersions aqueuses colloïdales de départ ou du rapport molaire acide organique/composé de $M^{n+}$.

Généralement, le diamètre hydrodynamique des colloïdes obtenus en milieu organique déterminé par diffusion quasi-élastique de la lumière est compris entre 8 nm (80 Å) et 120 nm (1200 Å).

Pour certaines applications il est possible d'utiliser le mélange réactionnel tel quel mais parfois il est souhaitable d'éliminer l'eau qui peut représenter de 1 à 3 % en poids de la phase organique. A cet effet, on met en oeuvre les moyens bien connus par l'homme de l'art, par exemple l'adjonction d'un tiers solvant inerte par rapport au composé de $M^{n+}$, ayant un point d'ébullition, de préférence, inférieur à 100°C et formant un azéotrope avec l'eau puis distillation de l'azéotrope obtenu. A titre de tiers solvants convenant à l'invention, on peut citer les hydrocarbures aliphatiques, tels que l'hexane, l'heptane; les hydrocarbures cycloaliphatiques: aromatiques; ou les alcools tels que par exemple, éthanol, éthylèneglycol, diéthylèneglycol etc..

L'invention s'applique tout particulièrement bien à la préparation de dispersions organiques colloidales des composés de cérium IV.

Lesdits sols obtenus selon l'invention présentent une concentration en composé de cérium IV qui peut être très élevée puisqu'elle peut aller jusqu'à 3,5 M à 4 M de $CeO_2$.

On constate que le rendement d'extraction du cérium dans la phase organique est très bon puisqu'il peut atteindre 90 à 95 %.

Par diffusion quasi élastique de la lumière, on met en évidence la présence de colloïdes ayant un diamètre hydrodynamique variable avec les conditions de préparation de l'ordre de 6 nm (60 Å) à 50 nm (500 Å).

Après insolubilisation des colloïdes par extraction du solvant organique dans l'acétone, on récupère une poudre dont l'analyse élémentaire montre un rapport molaire acide organique/cérium variant de 0,2 à 0,6 selon le mode d'obtention.

Les sols organiques ainsi élaborés présentent une excellente stabilité. On n'observe pas de décantation au bout de plusieurs mois.

L'invention concerne également l'utilisation des sols organiques préparés selon l'invention comme siccatifs dans l'industrie des peintures et vernis en vue d'accélérer le séchage des huiles insaturées et comme adjuvants de combustion dans les combustibles ou carburants liquides des générateurs énergétiques tels que moteurs à explosion, brûleurs à mazout ou propulseurs à réaction.

Pour permettre de mieux illustrer la mise en oeuvre de l'invention, on donne ci-après différents exemples, bien entendu non limitatifs.

Les exemples 1 à 5 concernent la préparation d'un sol organique de cérium IV dans différents milieux organiques.

Les exemples 6 et 7 sont relatifs à l'obtention d'un sol organique mixte de cérium IV et de fer III.

**Exemple 1**

1°) <u>Préparation d'une dispersion colloïdale aqueuse d'un composé de cérium IV</u>

Dans un ballon tricol de 6 litres muni d'un thermomètre, d'un dispositif d'agitation, d'un système d'introduction de réactif (pompe doseuse), on introduit à température ambiante, 1000 cm³ d'une solution de nitrate cérique contenant 1,64 moles/litre de cérium IV, 0,07 mole/litre de cérium III et ayant une acidité libre de 0,42 N obtenue par électrolyse conformément à FR-A-2 570 087.

Dans ladite solution maintenue sous agitation, on additionne à température ambiante, progressivement, 545 cm³ d'une solution d'ammoniaque 11,3 N à raison de 100 cm³/heure.

On obtient une dispersion colloïdale aqueuse d'un composé de cérium IV à une concentration exprimée en $CeO_2$ également à 183 g/l et un pH de 1,3.

On caractérise la taille des colloïdes par diffusion quasi-élastique de la lumière selon la méthode décrite par Michal L. McConnell dans Analytical Chemistry, Vol. 53 n° 8 1007 A (1981). Le diamètre moyen hydrodynamique des colloïdes est de l'ordre de 8 nm (80 Å).

2°) <u>Préparation d'un sol organique d'un composé de cérium IV</u>

Dans un erlenmeyer de 250 cm³ équipé d'un dispositif d'agitation magnétique, surmonté d'un réfrigérant ascendant et muni d'un dispositif de chauffage avec contrôle de température, on introduit 100 g du sol aqueux préparé selon a- et une phase organique comprenant 13,6 cm³ de méthylisobutylcétone et 16,8 cm³ d'acide oléique.

Le mélange est agité et porté à 90°C en maintenant cette température pendant 4 heures tout en agitant.

On observe, en fin de réaction, la formation d'une phase brune caractéristique de la dispersion colloïdale organique d'hydroxyde cérique.

On réalise la séparation de la phase organique et de la phase aqueuse par simple décantation.

On détermine une teneur en $CeO_2$ de l'ordre de 517 g/l dans le sol organique obtenu selon une technique qui consiste à calciner à 1000°C une prise d'essai du sol organique, puis à peser le résidu solide obtenu.

On met en évidence par diffusion quasi-élastique de la lumière la présence de colloïdes ayant un diamètre hydrodynamique de l'ordre de 10,9 nm (109 Å).

Après insolubilisation des colloïdes par extraction du solvant organique dans l'acétone, on récupère une poudre de coloration brun-marron. L'analyse élémentaire de cette poudre révèle un rapport molaire acide oléique/$CeO_2$ de l'ordre de 0,57: $CeO_2$ est déterminé par pesée après calcination et l'acide oléique par dosage par spectrométrie I.R. du gaz carbonique dégagé lors de la calcination.

**Exemple 2**

Dans le même type d'appareillage tel que décrit dans l'exemple 1, on met en contact 100 cm³ du sol aqueux préparé selon a- avec une phase organique comprenant 20,11 cm³ d'un hydrocarbure aromatique commercialisé sous la marque SOLVESSO 150 et 16,8 cm³ d'acide oléique.

Le mélange est agité et porté à 90°C: on maintient cette température pendant 4 heures tout en agitant.

Au bout de 2 heures, on observe la formation d'une phase brune caractéristique de la dispersion colloïdale organique d'hydroxyde cérique.

Après séparation des deux phases par décantation, on réalise un entraînement azéotropique de l'eau à l'hexane.

On dose une teneur en $CeO_2$ de l'ordre de 450 g/l dans le sol organique obtenu.

On détermine par diffusion quasi-élastique de la lumière un diamètre hydrodynamique des colloïdes de 15 nm (150 Å).

Le dosage chimique effectué sur la poudre après insolubilisation des colloïdes par extraction du solvant organique dans l'acétone montre un rapport molaire acide oléique $CeO_2$ de 0,5.

## Exemple 3

On met en contact 100 cm$^3$ du sol aqueux préparé selon a- de l'exemple 1 avec une phase organique comprenant 32 cm$^3$ d'un hydrocarbure aromatique commercialisé sous la marque SOLVESSO 150 et 8,4 cm$^3$ d'acide oléique.

Le mélange est agité et porté à 90°C: on maintient cette température pendant 24 heures tout en agitant.

Au bout de 12 heures, on observe la formation d'une phase brune caractéristique de la dispersion colloïdale organique d'hydroxyde cérique.

Après séparation des deux phases par décantation, on réalise un entraînement azéotropique de l'eau à l'hexane.

On dose une teneur en $CeO_2$ de l'ordre de 420 g/l dans le sol organique obtenu.

On détermine par diffusion quasi-élastique de la lumière un diamètre hydrodynamique des colloïdes du sol organique de 50 nm (500 Å).

Le dosage chimique effectué sur la poudre après insolubilisation des colloïdes par extraction du solvant organique dans l'acétone montre un rapport acide oléique $CeO_2$ de 0,31.

## Exemple 4

Dans le même type d'appareillage tel que décrit précédemment, on met en contact 100 cm$^3$ d'une dispersion colloïdale aqueuse d'un composé de cérium IV préparé selon le mode opératoire de l'exemple 1 contenant 0,7 mole/litre de cérium IV et ayant un pH de 1,8, avec une phase organique comprenant 15 cm$^3$ d'un solvant aliphatique commercialisé sous la marque AMSCO et 10,6 cm$^3$ d'acide oléique.

Le mélange est agité et porté à 90°C. On maintient cette température pendant 4 heures tout en agitant.

Au bout de 3 heures on observe la formation d'une phase brune caractéristique de la dispersion colloïdale organique d'hydroxyde cérique.

Après séparation de 2 phases par décantation, on réalise un entraînement azéotropique de l'eau à l'hexane.

On dose une teneur en $CeO_2$ de l'ordre de 300 g/l dans le sol organique obtenu.

On détermine par diffusion quasi-élastique de la lumière un diamètre hydrodynamique des colloïdes du sol organique de l'ordre de 15 nm (150 Å).

## Exemple 5

Dans le même type d'appareillage tel que décrit précédemment on met en contact 100 cm$^3$ d'une dispersion colloïdale aqueuse d'un composé de cérium IV préparé selon le mode opératoire de l'exemple 1 contenant 0,95 mole/litre de cérium IV et ayant un pH de 1,9 avec une phase organique comprenant 21,4 cm$^3$ de SOLVESSO 150, 15,2 cm$^3$ d'acide oléique et 1,1 cm$^3$ d'éthyl-2 hexanol.

Le mélange est agité et porté à 90°C. On maintient cette température pendant 4 heures tout en agitant.

Après séparation des 2 phases, on réalise un entraînement azéotropique de l'eau à l'hexane.

On dose une teneur de l'ordre de 375 g/l en $CeO_2$ dans le sol organique obtenu.

On détermine par diffusion quasi-élastique de la lumière un diamètre hydrodynamique des colloïdes du sol organique de l'ordre de 15 nm (150 Å).

## Exemple 6

On réalise une dispersion sursaturée en ions OH⁻ colloïdale de nitrate ferrique en additionnant à température ambiante 250 cm$^3$ d'ammoniaque 2,89 N à raison de 100 cm$^3$/h dans 250 cm$^3$ de solution de nitrate ferrique $Fe^{3+} = 1$ M et $H^+ = 0,5$ N.

Après 10 jours à température ambiante la solution colloïdale obtenue, présente un pH de 1,75 et une concentration de 0,5 M en $Fe^{3+}$.

On mélange 100 cm$^3$ de la dispersion sursaturée en ions OH⁻ colloïdale aqueuse d'hydroxyde ferrique précédemment synthétisée à 100 cm$^3$ de la solution colloïdale aqueuse d'hydroxyde cérique décrite à l'exemple 1 et on met au contact ce mélange avec 38,46 cm$^3$ d'acide oléique et 16,5 cm$^3$ de solvant SOLVESSO 150 à 90°C.

Au bout de 24 heures on recueille une phase brune par décantation et on réalise l'élimination de l'eau par entraînement azéotropique à l'hexane.

On détermine par diffusion quasi-élastique de la lumière un diamètre hydrodynamique des colloïdes du sol organique de l'ordre de 120 nm (1200 Å).

Par calcination à 1000°C, pendant 2 heures d'une partie aliquote de sol organique bien définie, on détermine une teneur d'environ 300 g/l en oxydes du sol organique synthétisé.

La teneur en cérium et en fer des colloïdes du sol organique synthétisé est déterminée par analyse fluorescence X sur le résidu solide obtenu après ultracentrifugation, séchage et calcination à 1000°C.

Le rapport molaire Fe/Ce déterminé comparativement à des étalons de composition Fe/Ce définie est de l'ordre de 0,5.

## Exemple 7

On réalise une dispersion sursaturée en ions $OH^-$ colloïdale de nitrate ferrique en additionnant à température ambiante 857 cm³ d'ammoniaque 6,65 N à raison de 100 cm³/h dans 2 litres de solution de nitrate ferrique $Fe^{3+}$ = 1 M et $H^+$ = 0,5 N.

La solution colloïdale obtenue présente un pH de 1,75 et une concentration de 0,70 M en $Fe^{3+}$.

On mélange 100 cm³ de la dispersion sursaturée en ions $OH^-$ colloïdale aqueuse d'hydroxyde ferrique précédemment synthétisée à 100 cm³ d'une solution colloïdale aqueuse d'hydroxyde cérique ($CeO_2$ = 120 g/l, pH = 1,8) et on met en contact ce mélange avec 21,6 cm³ d'acide oléique et 14,5 cm³ de solvant SOLVESSO 150 à 90°C.

Au bout de 24 heures on recueille une phase brune par décantation et on réalise l'élimination de l'eau par entraînement azéotropique à l'hexane.

On détermine par diffusion quasi-élastique de la lumière un diamètre hydrodynamique des colloïdes du sol organique de l'ordre de 90 nm (900 Å).

Par calcination à 1000°C pendant 2 heures d'une partie aliquote du sol organique on détermine une teneur d'environ 300 g/l en oxydes du sol organique synthétisé.

L'analyse par fluorescence X du produit calciné effectuée par rapport à des étalons de composition Fe/Ce définie montre un rapport molaire Fe/Ce de l'ordre de 1.

## Revendications

1. Procédé de préparation d'une dispersion colloïdale d'un composé d'un cation métallique à caractère acide en milieu organique caractérisé par le fait qu'il consiste:

- à mettre en contact:
   (a) une phase aqueuse constituée par au moins une dispersion colloïdale d'au moins un composé d'un cation métallique à caractère acide $M^{n+}$ en milieu aqueux sursaturé en ions $OH^-$ obtenue par réaction d'une solution aqueuse d'un sel du cation $M^{n+}$ et d'une base introduite en quantité telle qu'elle représente jusqu'à 95 % en moles de la quantité de base théorique nécessaire pour obtenir $M(OH)_n$,
   (b) une phase organique comprenant un acide organique et un milieu liquide organique ou solvant
- puis à séparer la phase aqueuse et la phase organique.

2. Procédé selon la revendication 1 caractérisé par le fait que la quantité de base représente de 60 à 95 % de la quantité de base théorique.

3. Procédé selon les revendications 1 et 2 caractérisé par le fait que le cation $M^{n+}$ est le cation des métaux suivants: cérium, fer, titane, zirconium, étain.

4. Procédé selon la revendication 3 caractérisé par le fait que le cation $M^{n+}$ est le cérium IV.

5. Procédé selon la revendication 1 caractérisé par le fait que le taux de métal M sous forme colloïdale dans le sol aqueux est supérieur ou égal à 95 %.

6. Procédé selon la revendication 1 caractérisé par le fait que la concentration du sol aqueux en composé de cation $M^{n+}$ varie entre 0,1 et 3 moles/litre.

7. Procédé selon la revendication 1 caractérisé par le fait que l'on prépare une dispersion colloïdale d'un composé de cérium IV en milieu aqueux sursaturée en ions $OH^-$ en faisant réagir une solution aqueuse d'un sel de cérium IV avec une base de façon à obtenir un taux de sursaturation supérieur à 3 et inférieur à 4.

8. Procédé selon la revendication 7 caractérisé par le fait que le taux de sursaturation est supérieur à 3 et inférieur ou égal à 3,8.

9. Procédé selon l'une des revendications 7 et 8 caractérisé par le fait que la solution aqueuse de sel de cérium IV est une solution aqueuse de nitrate cérique ou de nitrate céri-ammoniacal.

10. Procédé selon la revendication 1 caractérisé par le fait que l'on prépare une dispersion colloïdale d'un composé de fer III en milieu aqueux sursaturée en ions $OH^-$ en faisant réagir une solution aqueuse d'un sel de fer avec une base de façon à obtenir un taux de sursaturation supérieur à 2,2 et inférieur à 2,7.

11. Procédé selon la revendication 10 caractérisé par le fait que la solution aqueuse de sel de fer est une solution aqueuse de chlorure ou nitrate ferrique.

12. Procédé selon la revendication 10 caractérisé par le fait que la solution aqueuse de sel de fer est une solution aqueuse de chlorure ou nitrate ferreux et que l'on ajoute un agent oxydant.

13. Procédé selon l'une des revendications 1 à 12 caractérisé par le fait que l'on met en oeuvre deux ou plusieurs dispersions colloïdales aqueuses de différents composés d'un cation $M^{n+}$.

14. Procédé selon la revendication 13 caractérisé par le fait que l'on met en oeuvre une dispersion colloïdale d'un composé de cérium IV et une dispersion colloïdale d'un composé de fer III.

15. Procédé selon la revendication 14 caractérisé par le fait que la proportion entre lesdites dispersions est telle que l'on obtienne un mélange comprenant:

- de 15 à 85 % d'un composé de cérium IV exprimé en poids de cérium rapporté aux poids totaux des métaux,
- de 15 à 85 % d'un composé de fer III exprimé en poids de fer rapporté aux poids totaux des métaux.

16. Procédé selon la revendication 1 caractérisé par le fait que l'on prépare une dispersion colloïdale aqueuse d'un composé de différents cations $M^{n+}$ en faisant réagir une solution aqueuse d'au moins deux sels de métal M avec une base en une quantité de base telle qu'elle représente de 60 à 95 % en moles de la quantité de base théorique nécessaire à la neutralisation complète des cations $M^{n+}$ présents dans le milieu réactionnel pour obtenir $M(OH)_n$.

17. Procédé selon l'une des revendications 1 à 16 caractérisé par le fait que le milieu liquide organique est un hydrocarbure aliphatique ou cycloaliphatique ou leur mélange, un hydrocarbure aromatique, un hydrocarbure aromatique chloré, un éther aliphatique ou cycloaliphatique, une cétone aliphatique ou cycloaliphatique.

18. Procédé selon la revendication 17 caractérisé par le fait que le milieu liquide organique est l'hexane, l'heptane, l'octane, le nonane, le décane, le cyclohexane, le cyclopentane, le cycloheptane, les naphtènes liquides, les essences minérales ou de pétrole, éthers minéraux ou de pétrole; le benzène, le toluène, les xylènes; le chloro- ou dichlorobenzène et le chlorotoluène; l'éther de disopropyle, l'éther de dibutyle, la méthylisobutylcétone, la diisobutylcétone, l'oxyde de mésityle.

19. Procédé selon l'une des revendications 1 à 18 caractérisé par le fait que l'on ajoute dans la phase organique un agent promoteur.

20. Procédé selon la revendication 19 caractérisé par le fait que l'agent promoteur est un alcool aliphatique linéaire ou ramifié ayant de 6 à 12 atomes de carbone.

21. Procédé selon la revendication 20 caractérisé par le fait que l'agent promoteur est l'éthyl-2 hexanol, le décanol, le dodécanol ou un mélange d'entre eux.

22. Procédé selon l'une des revendications 1 à 21 caractérisé par le fait que l'acide organique est un acide ou un mélange d'acides naturels ou synthétiques tels que les acides carboxyliques aliphatiques, les acides sulfoniques aliphatiques, les acides phosphoniques aliphatiques, les acides alcoylarylsulfoniques, les acides alcoylarylphosphoniques.

23. Procédé selon la revendication 22 caractérisé par le fait que l'acide organique est un acide ou un mélange d'acides tels que les acides gras de tallöl, d'huile de coco, de soja, de suif, d'huile de lin, l'acide oléique, l'acide linoléique, l'acide stéarique, l'acide isostéarique, l'acide pelargonique, l'acide caprique, l'acide laurique, l'acide myristique, l'acide dodécylbenzènesulfonique, l'acide éthyl-2 hexoïque, l'acide naphténique, l'acide hexoïque, l'acide toluène-sulfonique, l'acide toluène-phosphonique, l'acide lauryl-sulfonique, l'acide lauryl-phosphonique, l'acide palmityl-sulfonique et l'acide palmityl-phosphonique.

24. Procédé selon la revendication 23 caractérisé par le fait que l'acide organique est un acide ou un mélange d'acides suivants: acide oléïque, acide dodécyl-benzènesulfonique.

25. Procédé selon la revendication 1 caractérisé par le fait que la quantité d'acide organique varie dans l'intervalle de 0,25 à 1 mole par mole de $M_2O_n$.

26. Procédé selon la revendication 25 caractérisé par le fait que ladite quantité est de 0,25 à 0,8 mole par mole de $M_2O_n$.

27. Procédé selon la revendication 1 caractérisé par le fait que le rapport pondéral entre le solvant organique est l'acide organique est compris entre 0,3 et 2,0.

28. Procédé selon l'une des revendications 1 à 27 caractérisé par le fait que l'on mélange la ou les dispersions aqueuses colloïdales, l'acide organique, le solvant organique et éventuellement l'agent promoteur.

29. Procédé selon l'une des revendications 1 à 27 caractérisé par le fait que l'on prémélange l'acide organique, le solvant organique et éventuellement l'agent promoteur avant d'effectuer le mélange de la phase organique obtenue avec la dispersion aqueuse colloïdale.

30. Procédé selon l'une des revendications 1 à 29 caractérisé par le fait que l'on chauffe le mélange réactionnel à une température variant entre 60 et 150°C.

31. Procédé selon la revendication 30 caractérisé par le fait que ladite température est comprise entre 80 et 100°C.

**Patentansprüche**

1. Verfahren zur Herstellung einer kolloidalen Dispersion einer Verbindung eines Metallkations mit saurem Charakter in organischem Milieu, dadurch gekennzeichnet, daß es darin besteht:
- (a) eine wässrige Phase bestehend aus wenigstens einer kolloidalen Dispersion wenigstens einer Verbindung eines Metallkations mit saurem Charakter $M^{n+}$ in wässrigem Milieu, das mit $OH^-$-Ionen übersättigt ist, erhältlich durch Reaktion einer wässrigen Lösung eines Salz es des Kations $M^{n+}$ und einer Base, die in einer solchen Menge eingebracht wird, daß sie bis zu 95 Mol.-% der theoretischen Menge darstellt, die notwendig ist, um $M(OH)_n$ zu erhalten,

in Kontakt zu bringen mit:

(b) einer organischen Phase enthaltend eine organische Säure und ein flüssiges organisches Medium oder Solvens, - und dann die wässrige Phase und die organische Phase zu trennen.

2. Verfahren nach Anspruch 1, dadurch gekennzeichnet, daß die Menge der Base 60 bis 95 % der theoretischen Basenmenge darstellt.

3. Verfahren nach den Ansprüchen 1 und 2, dadurch gekennzeichnet, daß das Kation $M^{n+}$ das Kation der nachfolgenden Metalle ist: Cer, Eisen, Titan, Zirkonium, Zinn.

4. Verfahren nach Anspruch 3, dadurch gekennzeichnet, daß das Kation $M^{n+}$ Cer IV ist.

5. Verfahren nach Anspruch 1, dadurch gekennzeichnet, daß der Anteil an Metall M in kolloidaler Form in dem wässrigen Sol größer oder gleich 95 % ist.

6. Verfahren nach Anspruch 1, dadurch gekennzeichnet, daß die Konzentration des wässrigen Sols an der Verbindung des Kations $M^{n+}$ von 0,1 bis 3 Mol pro Liter variiert.

7. Verfahren nach Anspruch 1, dadurch gekennzeichnet, daß man eine kolloidale Dispersion einer Verbindung des Cer IV in wässrigem Milieu, das an $OH^-$-Ionen übersättigt ist, herstellt, indem man eine wässrige Lösung eines Cer IV-Salzes mit einer Base in der Weise reagieren läßt, daß man einen Übersättigungsgrad von größer als 3 und geringer als 4 erhält.

8. Verfahren nach Anspruch 7, dadurch gekennzeichnet, daß der Übersättigungsgrad größer als 3 und kleiner oder gleich 3,8 ist.

9. Verfahren nach einem der Ansprüche 7 und 8, dadurch gekennzeichnet, daß die wässrige Lösung des CerIV-Salzes eine wässrige Lösung von Cernitrat oder Cerammonnitrat ist.

10. Verfahren nach Anspruch 1, dadurch gekennzeichnet, daß man eine kolloidale Dispersion einer Verbindung des Eisen III in wässrigem Milieu herstellt, das an $OH^-$-Ionen übersättigt ist, indem man eine wässrige Lösung eines Eisensalzes mit einer Base in der Weise reagieren läßt, daß man einen Übersättigungsgrad von größer als 2,2 und kleiner als 2,7 erhält.

11. Verfahren nach Anspruch 10, dadurch gekennzeichnet, daß die wässrige Lösung des Eisensalzes eine wässrige Lösung von Eisenchlorid oder Eisennitrat ist.

12. Verfahren nach Anspruch 10, dadurch gekennzeichnet, daß die wässrige Lösung des Eisensalzes eine wässrige Lösung von Eisen(II)-chlorid oder -nitrat ist und man ein Oxidationsmittel zugibt.

13. Verfahren nach einem der Ansprüche 1 bis 12, dadurch gekennzeichnet, daß man zwei oder mehrere kolloidale wässrige Dispersionen verschiedener Verbindungen eines Kations $M^{n+}$ einsetzt.

14. Verfahren nach Anspruch 13, dadurch gekennzeichnet, daß man eine kolloidale Dispersion einer Verbindung des Cer IV und eine kolloidale Dispersion einer Eisen(III)-Verbindung einsetzt.

15. Verfahren nach Anspruch 14, dadurch gekennzeichnet, daß das Verhältnis zwischen diesen Dispersionen so ist, daß man ein Gemisch erhält enthaltend:

- 15 bis 85 % einer CerIV-Verbindung ausgedrückt in Gewicht des Cers bezogen auf das Gesamtgewicht der Metalle,

-15 bis 85 % einer Verbindung des Eisen (III) ausgedrückt als Gewicht des Eisens bezogen auf das Gesamtgewicht der Metalle.

16. Verfahren nach Anspruch 1, dadurch gekennzeichnet, daß man eine kolloidale wässrige Dispersion einer Verbindung verschiedener Kationen $M^{n+}$ herstellt, indem man eine wässrige Lösung wenigstens zweier Salze des Metalls M mit einer Base reagieren läßt, mit einer solchen Basenmenge, daß diese 60 bis 95 Mol.-% der theoretischen Basenmenge darstellt, die notwendig ist, um die in dem Reaktionsmedium anwesenden Kationen $M^{n+}$ vollständig zu neutralisieren und um $M(OH)_n$ zu erhalten.

17. Verfahren nach einem der Ansprüche 1 bis 16, dadurch gekennzeichnet, daß das flüssige organische Milieu ein aliphatischer oder cycloaliphatischer Kohlenwasserstoff oder deren Gemisch ist, ein aromatischer Kohlenwasserstoff, ein chlorierter aromatischer Kohlenwasserstoff, ein aliphatischer oder cycloaliphatischer Ether, ein aliphatisches oder cycloaliphatisches Keton.

18. Verfahren nach Anspruch 17, dadurch gekennzeichnet, daß das flüssige organische Milieu Hexan, Heptan, Octan, Nonan, Decan, Cyclohexan, Cyclopentan, Cycloheptan, ein flüssiges Naphthen, ein Mineralöl oder Benzin, ein Mineralether oder Petrolether, Benzol, Toluol, ein Xylol, Chlorbenzol oder Dichlorbenzol, Chlortoluol, Diisopropylether, Dibutylether, Methylisobutylketon, Diisobutylketon, Mesityloxid ist.

19. Verfahren nach einem der Ansprüche 1 bis 18, dadurch gekennzeichnet, daß man zu der organischen Phase ein Beschleunigungsreagenz zugibt.

20. Verfahren nach Anspruch 19, dadurch gekennzeichnet, daß das Beschleunigungsreagenz ein linearer oder verzweigter aliphatischer Alkohol mit 6 bis 12 Kohlenstoffatomen ist.

21. Verfahren nach Anspruch 20, dadurch gekennzeichnet, daß das Beschleunigungsreagenz 2-Ethylhexanol, Decanol, Dodecanol oder ein Gemisch von diesen ist.

22. Verfahren nach einem der Ansprüche 1 bis 21, dadurch gekennzeichnet, daß die organische Säure eine natürliche oder synthetische Säure oder ein Gemisch natürlicher oder synthetischer Säuren ist, wie zum Beispiel aliphatische Carbonsäuren, aliphatische Sulfonsäuren, aliphatische Phosphonsäuren, Alkylarylsulfonsäuren, Alkylarylphosphonsäuren.

23. Verfahren nach Anspruch 22, dadurch gekennzeichnet, daß die organische Säure eine Säure oder ein Gemisch von Säuren wie zum Beispiel Fettsäuren des Tallöls, Kokosöls, des Soja, des Talks, des Leinöls, Ölsäure, Linolsäure, Stearinsäure, Isostearinsäure, Pelargonsäure, Caprinsäure, Laurinsäure, Myristinsäure, Dodecylbenzolsulfonsäure, 2-Ethylhexansäure, Naphthensäure, Hexansäure, Toluolsulfonsäure, Toluolphosphonsäure, Laurylsulfonsäure, Laurylphosphonsäure, Palmitylsulfonsäure, Palmitylphosphonsäure.

24. Verfahren nach Anspruch 23, dadurch gekennzeichnet, daß die organische Säure eine der nachfolgenden Säuren oder ein Gemisch der nachfolgenden Säuren ist: Ölsäure, Dodecylbenzolsulfonsäure.

25. Verfahren nach Anspruch 1, dadurch gekennzeichnet, daß die Menge der organischen Säure im Intervall von 0,25 bis 1 Mol pro Mol $M_2O_n$ variiert.

26. Verfahren nach Anspruch 25, dadurch gekennzeichnet, daß diese Menge von 0,25 bis 0,8 Mol pro Mol $M_2O_n$ beträgt.

27. Verfahren nach Anspruch 1, dadurch gekennzeichnet, daß das Gewichtsverhältnis zwischen organischem Solvens und organischer Säure zwischen 0,3 und 2,0 liegt.

28. Verfahren nach einem der Ansprüche 1 bis 27, dadurch gekennzeichnet, daß man die wässrige(n) kolloidale(n) Dispersion(en), die organische Säure, das organische Solvens und gegebenenfalls das Beschleunigungsreagenz mischt.

29. Verfahren nach einem der Ansprüche 1 bis 27, dadurch gekennzeichnet, daß man zunächst die organische Säure, das organische Solvens und gegebenenfalls das Beschleunigungsreagenz mischt bevor man die Mischung der erhaltenen organischen Phase mit der wässrigen kolloidalen Dispersion vornimmt.

30. Verfahren nach einem der Ansprüche 1 bis 29, dadurch gekennzeichnet, daß man das Reaktionsmedium auf eine Temperatur zwischen 60 und 150°C erwärmt.

31. Verfahren nach Anspruch 30, dadurch gekennzeichnet, daß diese Temperatur zwischen 80 und 100°C liegt.

## Claims

1. Process for the preparation of a colloidal dispersion of a compound of a metal cation of an acidic nature in an organic medium, characterized in that it consists:
- in bringing into contact:
(a) an aqueous phase consisting of at least one colloidal dispersion of at least one compound of a metal cation of an acidic nature $M^{n+}$ in an aqueous medium supersaturated with $OH^-$ ions, obtained by reaction of an aqueous solution of a salt of the cation $M^{n+}$ and of a base introduced in such quantity that it represents up to 95 mol-% of the theoretical quantity of base required to obtain $M(OH)_n$,
(b) an organic phase comprising an organic acid and an organic liquid medium or solvent,
- and in then separating the aqueous phase from the organic phase.

2. Process according to Claim 1, characterized in that the quantity of base represents from 60 to 95 % of the theoretical quantity of base.

3. Process according to Claims 1 and 2, characterized in that the cation $M^{n+}$ is the cat ion of the following metals: cerium, iron, titanium, zirconium, tin.

4. Process according to Claim 3, characterized in that the cation $M^{n+}$ cerium (IV).

5. Process according to Claim 1, characterized in that the proportion of metal M in colloidal form in the aqueous sol is greater than or equal to 95 %.

6. Process according to Claim 1, characterized in that the concentration of the compound of cation $M^{n+}$ in the aqueous sol varies between 0.1 and 3 moles/litre.

7. Process according to Claim 1, characterized in that a colloidal dispersion of a cerium (IV) compound in an aqueous medium, supersaturated with $OH^-$ ions, is prepared by reacting an aqueous solution of a cerium (IV) salt with a base so as to obtain a degree of supersaturation greater than 3 and smaller than 4.

8. Process according to Claim 7, characterized in that the degree of supersaturation is greater than 3 and smaller than or equal to 3.8.

9. Process according to either of Claims 7 and 8, characterized in that the aqueous solution of cerium (IV) salt is an aqueous solution of ceric nitrate or of ceric ammonium nitrate.

10. Process according to Claim 1, characterized in that a colloidal dispersion of an iron (III) compound in an aqueous medium, supersaturated with $OH^-$ ions is prepared by reacting an aqueous solution of an iron salt with a base so as to obtain a degree of supersaturation greater than 2.2 and smaller than 2.7.

11. Process according to Claim 10, characterized in that the aqueous iron salt solution is an aqueous solution of ferric chloride or nitrate.

12. Process according to Claim 10, characterized in that the aqueous iron salt solution is an aqueous solution of ferrous chloride or nitrate and in that an oxidizing agent is added.

13. Process according to one of Claims 1 to 12, characterized in that two or more aqueous colloidal dispersions of different compounds of a cation $M^{n+}$ are used.

14. Process according to Claim 13, characterized in that a colloidal dispersion of a cerium (IV) compound and a colloidal dispersion of an iron (III) compound are used.

13

15. Process according to Claim 14, characterized in that the proportion between the said dispersions is such that a mixture comprising:

- from 15 to 85 % of a cerium (IV) compound expressed as a weight of cerium relative to the total weights of metals,
- from 15 to 85 % of an iron (III) compound expressed as a weight of iron relative to the total weights of metals is obtained.

16. Process according to Claim 1, characterized in that an aqueous colloidal dispersion of a compound of various cations $M^{n+}$ prepared by reacting an aqueous solution of at least two salts of metal M with a base in a quantity of base such that it represents from 60 to 95 mol-% of the theoretical quantity of base required for complete neutralization of the cations $M^{n+}$ which are present in the reaction medium to obtain $M(OH)n$.

17. Process according to one of Claims 1 to 16, characterized in that the organic liquid medium is an aliphatic or cycloaliphatic hydrocarbon or a mixture thereof, an aromatic hydrocarbon, a chlorinated aromatic hydrocarbon, an aliphatic or cycloaliphatic ether or an aliphatic or cycloaliphatic ketone.

18. Process according to Claim 17, characterized in that the organic liquid medium is hexane, heptane, octane, nonane, decane, cyclohexane, cyclopentane, cycloheptane, liquid naphthenes, mineral or petroleum spirits, mineral or petroleum ethers, benzene, toluene, xylenes, chloroor dichlorobenzene and chlorotoluene, diisopropyl ether, dibutyl ether, methyl isobutyl ketone, diisobutyl ketone or mesityl oxide.

19. Process according to one of Claims 1 to 18, characterized in that a promoting agent is added into the organic phase.

20. Process according to Claim 19, characterized in that the promoting agent is a linear or branched aliphatic alcohol containing from 6 to 12 carbon atoms.

21. Process according to Claim 20, characterized in that the promoting agent is 2-ethylhexanol, decanol, dodecanol or a mixture thereof.

22. Process according to one of Claims 1 to 21, characterized in that the organic acid is an acid or a mixture of natural or synthetic acids such as aliphatic carboxylic acids, aliphatic sulphonic acids, aliphatic phosphonic acids, alkylarylsulphonic acids or alkylarylphosphonic acids.

23. Process according to Claim 22, characterized in that the organic acid is an acid or a mixture of acids such as the fatty acids from tall oil, coconut, soya or tallow oil, linseed oil, oleic acid, linoleic acid, stearic acid, isostearic acid, pelargonic acid, capric acid, lauric acid, myristic acid, dodecylbenzenesulphonic acid, 2-ethylhexoic acid, naphthenic acid, hexoic acid, toluenesulphonic acid, toluenephosphonic acid, laurylsulphonic acid, laurylphosphonic acid, palmitylsulphonic acid and palmitylphosphonic acid.

24. Process according to Claim 23, characterized in that the organic acid is an acid or a mixture of the folloving acids: oleic acid, dodecylbenzenesulphonic acid.

25. Process according to Claim 1, characterized in that the quantity of organic acid varies in the range from 0.25 to 1 mole per mole of $M_2O_n$.

26. Process according to Claim 25, characterized in that the said quantity is from 0.25 to 0.8 mole per mole of $M_2O_n$.

27. Process according to Claim 1, characterized in that the weight relationship between the organic solvent and the organic acid is between 0.3 and 2.0.

28. Process according to one of Claims 1 to 27, characterized in that the colloidal aqueous dispersion(s), the organic acid, the organic solvent and, if desired, the promoting agent are mixed.

29. Process according to one of Claims 1 to 27, characterized in that the organic acid, the organic solvent and, if desired, the promoting agent are premixed before mixing of the organic phase obtained with the colloidal aqueous dispersion is performed.

30. Process according to one of Claims 1 to 29, characterized in that the reaction mixture is heated to a temperature ranging between 60 and 150°C.

31. Process according to Claim 30, characterized in that the said temperature is between 80 and 100°C.